# EUROPÄISCHE PATENTSCHRIFT

(11) **EP 2 516 617 B1**
(45) Veröffentlichungstag und Bekanntmachung des Hinweises auf die Patenterteilung: **16.04.2014**
(21) Anmeldenummer: 10814664.8
(22) Anmeldetag: 20.12.2010
(51) Int. Cl.: C12M 1/107

(54) **FERMENTIERBEHÄLTER FÜR EINE BIOGASANLAGE**
FERMENTING TANK FOR A BIOGAS PLANT
CONTENANT DE FERMENTATION DESTINÉ À UNE INSTALLATION DE BIOGAZ

(30) Priorität: 22.12.2009 DE 102009059262
(43) Veröffentlichungstag der Anmeldung: 31.10.2012
(73) Patentinhaber: Dory, Thomas, 80801 München (DE); Unterlechner, Horst, 86899 Landsberg am Lech (DE)
(72) Erfinder: Dory, Thomas, 80801 München (DE); Unterlechner, Horst, 86899 Landsberg am Lech (DE)
(74) Vertreter: Moore, Joanne Camilla
(86) Internationale Anmeldenummer: PCT/IB2010/055957
(87) Internationale Veröffentlichungsnummer: WO 2011/077358

(56) Entgegenhaltungen:
- WO-A1-2008/102478
- CN-Y- 201 292 361
- CN-Y- 201 313 896
- DE-A1- 10 017 436
- DE-A1-102005 049 476
- DE-A1-102006 033 763
- DE-U1-202006 016 971
- NL-C2- 1 032 897

## Beschreibung

Die Erfindung betrifft einen Fermentierbehälter für eine Biogasanlage. Der Innenraum des Fermentierbehälters ist zumindest teilweise von mediendichten, druckfesten und thermisch isolierenden Wänden umgeben. Der Fermentierbehälter weist thermisch isolierende Außenwand-Bausegmente auf.

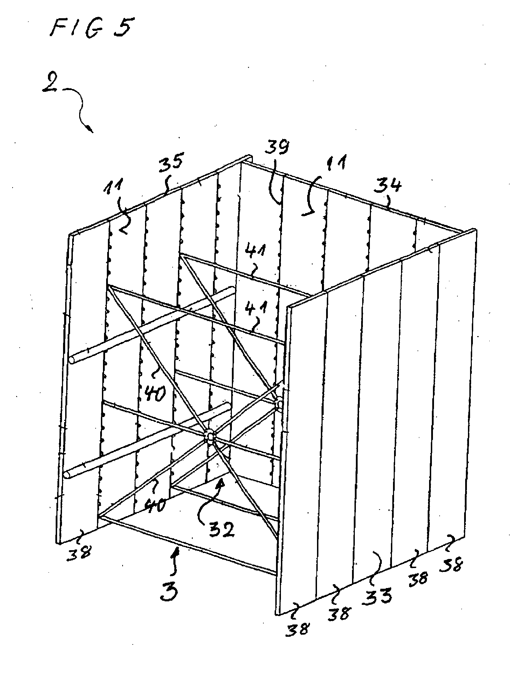

Ein derartiger Fermentierbehälter für Anlagen zur Erzeugung von Biogas ist aus der Druckschrift DE 10 2006 033 763 A1 bekannt. Der Innenraum dieses Fermentierbehälters ist zumindest teilweise von druckfesten, thermisch isolierenden Wänden umgeben, wie es die Figur 5 zeigt. Dazu weist mindestens eine Wand mindestens ein Paneel auf, das seinerseits zusammengesetzt ist aus einer Innenschale, die aus einem profilierten Blech hergestellt ist und auf der zum Inneren des Fermentierbehälters weisenden Seite der Wand angeordnet ist. Ferner weisen die isolierenden Wände eine Kassette auf, die einen Mantel aus Blech sowie einen von dem Mantel zumindest auf dessen nach innen zeigender Seite gehaltenen Isolierkörper aus thermisch isolierendem Material aufweist. Dabei ist die mindestens ein Paneel aufweisende Wand mit der gegenüberliegenden Wand durch mindestens ein Zugmittel verbunden, das den auf die beiden miteinander verbunden Wände wirkenden Innendruck des Behälters abfängt.

Figur 5 zeigt einen derartigen bekannten Fermentierbehälter 2 in einer teilweise aufgeschnittenen perspektivischen Ansicht, so dass der Innenraum 3 des Fermentierbehälters 2 sichtbar ist. Dieser Fennentierbehälter 2 ist aus hohen länglichen Paneelen 38 zusammengestellt. Dabei werden mehrere Paneelen 38 zu einer geraden Behälterwand zusammengefügt und von einem komplexen Innenaufbau 32 im Innenraum 3 werden die Außenwände 33, 34 und 35 sowie eine vierte hier nicht gezeigte Wand getragen und gestützt, so dass dieser Fermentierbehälter 2 mit viereckigem Querschnitt seinen Innenraum 3 beibehält, selbst wenn der Fermentierbehälter 2 in ein Erdreich eingebaut wird.

Ohne den stützenden komplexen Innenaufbau 32 würde der bekannte Fermentierbehälter 2 sich verwölben und gegebenenfalls zusammen- oder auseinanderbrechen. Nachteilig an dem bekannten Fermentierbehälter 2 ist darüber hinaus der komplexe In-nenaufbau mit Diagonalstreben 40 und Zug- und Druckstangen 41 und 42 zur Aufrechterhaltung der Form des Fermentierbehälters 2, so dass ein Reinigen in vorgeschriebenen Wartungsintervallen nicht ungefährlich ist, da der Bewegungsspielraum für das Wartungspersonal stark eingeengt ist. Ein weiterer Nachteil des bekannten Fermentierbehälters 2 ist die aufwendige Verbindung zwischen den einzelnen Segmenten bzw. Paneelen 38, welche die Außenseiten des rechteckförmigen Fermentierbehälters 2 bilden. Schließlich ist ein weiterer Nachteil, dass durch ein Verbinden mit in den Innenraum 3 hineinragenden Laschen 39 der Paneele 38 zu den Außenwänden 33, 34 und 35 des Behälters die Möglichkeit einer mediendichten Abdichtung durch eine entsprechende gummielastische Folie auf den Innenflächen 11 der Außenwände 33, 34 und 35 praktisch nicht möglich ist. Darüber hinaus sind die Verankerungen der Diagonalstreben 40 und der Zug- und Druckstangen 41 zwischen den Wänden 33 und 35 komplex und kostenintensiv.

DE 10 2007005069 offenbart eine Biogasgewinnungsanlage mit einem Nachfermenter. Die Fermenter liegen in einem Behälterverbund vor, wobei im Behälterverbund mit ringförmig geschlossenen Zwischenwänden wenigstens zwei ineinander liegende Behälterabteile ausgebildet sich. Die Ringformen können dabei rechteckig, quadratisch, oval, kreisrund oder in anderen unregelmäßigen (polygon) Formen gebildet sein. Die Behälter können konzentrisch angeordnet sein.

Aufgabe der Erfindung ist es, einen Fermentierbehälter zu schaffen, der die Nachteile im Stand der Technik überwindet und kostengünstig herstellbar ist.

Diese Aufgabe wird mit dem Gegenstand des unabhängigen Anspruchs 1 gelöst. Vorteilhafte Weiterbildungen der Erfindung ergeben sich aus den abhängigen Ansprüchen.

Erfindungsgemäß wird ein Fermentierbehälter für eine Biogasanlage geschaffen. Der Innenraum des Fermentierbehälters ist zumindest teilweise von mediendichten, druckfesten und thermisch isolierenden Wänden umgeben. Der Fermentierbehälter weist thermisch isolierende Außenwand-Leichtbausegmente auf. Die Außenwand-Leichtbausegmente bilden einen polygonalen selbst tragenden Ring.

Ein derartiger Fermentierbehälter für eine Biogasanlage hat den Vorteil, dass er kostengünstig durch ringförmiges Zusammenstellen vorgefertigter Außenwand-Leichtbausegmente aufgestellt und mediendicht abgedichtet werden kann. Der weitere Vorteil besteht darin, dass die isolierenden Außenwand-Leichtbausegmente kostengünstig transportiert und zusammengebaut werden können, ohne dass zusätzliche Stützstrukturen erforderlich sind, um den polygonalen Ring als Fermentierbehälter für eine Biogasanlage zu nutzen. Nach Zusammenbau dieses polygonalen selbst tragenden Ringes aus thermisch isolierenden Außenwand-Leichtbausegmenten auf einer eingeebneten Fläche kann nach Einbringen einer entsprechend mediendichten Schutzfolie auf die Innenseiten der Leichtbausegmente und auf den Boden des Fermentierbehälters das Einleiten von Fermentiersubstanzen beginnen und die Gewinnung des Biogases, das sich unter einer gasdichten Abdeckung der Oberseite des Fermentierbehälters ansammelt, gewonnen werden.

Ein weiterer Vorteil dieses Fermentierbehälters ist, dass auf jegliche Innenstruktur im Innenraum des Fermentierbehälters verzichtet werden kann, so dass die Wartungs-, Reinigungs- und Inspektionsarbeiten des Fermentierbehälters kostengünstig gegenüber dem oben gezeigten Fermentierbehälter gemäß dem Stand der Technik und ohne Zugangsschwierigkeiten zu den Innenflächen des polygonalen Ringes aus Außenwand-Leichtbausegmenten durchgeführt werden können.

In einer Ausführungsform der Erfindung weisen die Außenwand-Leichtbausegmente Wärmedämmplatten mit mindestens einer äußeren Deckschicht aus korrosionsgeschütztem Stahlblech auf. Dabei sorgt diese äußere Deckschicht aus korrosionsgeschütztem Stahlblech in vorteilhafter Weise für eine Formstabilität des polygonal selbst tragenden Ringes, während die Wärmedämmplatten mit einer Dicke von über 50 mm für die Aufrechterhaltung der Reaktionswärme in dem Fermentierbehälter sorgen.

In vorteilhafter Weise bestehen die Wärmedämmplatten aus geschlossenporigem Polyurethan, das aufgrund der Geschlossenporigkeit des Polyurethan-Schaums sich nicht wie ein Schwamm mit Wasser oder anderen Flüssigkeiten voll saugen kann und somit die Wärmedämmeigenschaften auch ohne Schutz vor Umwelteinflüssen beibehält. Anstelle des geschlossenporigen Polyurethan-Schaums kann auch Styropor eingesetzt werden, jedoch ist dessen mechanische Festigkeit und Formstabilität nicht so günstig wie die des Polyurethan-Schaums. Außerdem kann Polyurethan-Schaum in entsprechend vorbereiteten Hohlformen zu Wärmedämmplatten ausgebildet werden, ohne dass wie bei Polystyrol Druckformen und/oder Schneidwerkzeuge erforderlich sind.

Weiterhin ist es vorgesehen, dass die Ecken des polygonalen Ringes vertikal angeordnete Nut-Federübergänge der Wärmedämmplatten aufweisen. Diese Nut-Federübergänge aus dem Material der Wärmedämmplatten zeigen gegenüber dem Stand der Technik eine verbesserte Wärmeisolation, zumal dessen Eckstrukturen durch metallische Profilbleche realisiert sind, was einerseits die Kosten hochtreibt und andererseits eine Wärmesenke für die in dem Fermentierbehälter reagierende Biomasse darstellt.

Verbleibende senkrechte Fugen der Nut-Federübergänge der Wärmedämmplatten an den Ecken des polygonalen Ringes können ohne großen Zeitaufwand mit Polyurethan-Schaum aufgefüllt und mittels Abdeckblechen und einer Silikonzwischenschicht nach dem Aufstellen der ringförmig angeordneten Außenwand-Leichtbausegmente mediendicht geschlossen werden.

In einer weiteren Ausführungsform der Erfindung ist der polygonale selbst tragende Ring gegenüber einer Geländeoberfläche bis zu einer Mindesttiefe in die Erde eingelassen. Außerdem sind die Außenwand-Leichtbausegmente mit einer Drainagefolie und Abdichtungsfolie gegen auslaufendes Wasser und Fermentiersubstanzen aus einem Flies abgedeckt, wobei auf dem Flies eine Noppenfolie befestigt ist. Diese Noppenfolie hat den Vorteil einer verbesserten Wasser- und Flüssigkeitsableitung, so dass Wasser, Feuchte und Flüssigkeit über die Drainagefolie einer Drainageleitung, die rund um den polygonalen selbst tragenden Ring angeordnet ist, zugeführt werden können und mit Hilfe der Drainageableittung in einen Vorfluter der Biogasanlage eingeleitet werden können. In dem Vorfluter abgeschiedene Biomasse kann zu dem Fermentierbehälter zurückgeführt werden.

Von Vorteil ist es, wenn ein Mindesterdaushub von 2,5 m für ein Einlassen des polygonalen Ringes des Fermentierbehälters in der Erde vorgesehen ist. Entsprechend sind auch die Außendimensionen der Außenwand-Leichtbausegmente in Bezug auf ihre Breite b_{A} von mehreren 10 cm und ihre Höhe h_{A} von mehreren Metern sowie ihrer Dicke d_{A} von mehreren Zentimeter für die Realisierung eines derartigen polygonalen Außenringes vorgesehen.

Diese Außenwand-Leichtbausegmente sind als polygonaler selbst tragender Ring auf einer Bodenplatte unterhalb der Geländeoberfläche in einer Ausführungsform der Erfindung angeordnet, wobei zunächst eine Grundschicht aus unbewährtem Beton in einen entsprechenden Erdaushub eingebracht wird und schließlich ein mindestens 25 cm dicker Stahlbeton als Bodenplatte in dem Erdreich untergebracht wird. Ein auf einer derartigen Bodenplatte aufgebrachter polygonaler selbst tragender Ring weist für eine mediendichte Aufnahme von einer Biomasse eine mehrere Millimeter dicke mediendichte EPDM-Folie (Ethylen-Propylen-Dien-Kautschukfolie) auf, welche die Innenflächen der Außenwand-Leichtbausegmente bedeckt und auf der Bodenplatte liegend, diese mediendicht abdeckt. Dazu ist die EPDM-Folie mit den Innenflächen der Außenwand-Leichtbausegmente mittels einer Klebstoffschicht stoffschlüssig verbunden.

Diese zwischen 3 mm bis 5 mm dicke Kautschukfolie eines terpolymeren Elastomers weist eine hohe Wetter- und Feuchtigkeitsbeständigkeit und eine Ozonresistenz sowie eine hohe thermische Beständigkeit auf. Sie ist auch wegen ihrer hohen Elastizität und guten chemischen Beständigkeit als Flächendichtung und somit als Auskleidung in einer Ausführungsform des Fermentierbehälters eingesetzt. Eine besonders günstige Eigenschaft einer derartigen EPDM-Dichtungsfolie ist ihre Frostsicherheit, zumal sie bei Kälte hochflexibel bleibt und UV-beständig ist und somit langlebig ist. Durchdringungen der EPDM-Folie von Befestigungsmitteln können durch Flanschaufsätze abgedichtet sein. Rohrleitungsdurchdringungen können zusätzlich mit Klemmringen an den Rohren und Stützen gegen entsprechendes Abrutschen gesichert sein.

In einer weiteren Ausführungsform der Erfindung weist der Fermentierbehälter W and-Deckenübergänge auf, in denen in einem vorgegebenen Abstand Edelstahlwinkel angeordnet sind. Mit diesen Edelstahlwinkeln wird eine Behälterdecke mit der Wand verbunden und die EPDM-Folie wird zwischen Winkel und Wand gehalten. Dazu kann die EPDM-Folie zunächst mittels Klebstoff angeklebt werden und dann mit nicht rostenden Stahlschrauben durch die EPDM-Folie und an der inneren Deckschicht aus korrosionsgeschütztem Stahlblech fixiert werden. Um auch die Schenkel eines solchen Abdeckwinkels gegenüber der EPDM-Folie mediendicht abzudichten wird vorzugsweise eine Silikonschnur auf den Schenkeln der Abdeckwinkel vor dem Verschrauben angeordnet, so dass sich beim Anschrauben eine Silikonzwischenschicht ausbildet. Derartige Abdeckwinkel für die W and-Deckenverbindung sind vorzugsweise in Abständen von mehreren 10 cm vorgesehen.

In einer weiteren Ausführungsform der Erfindung ist eine polygonale ringförmige Trennwand aus Trennwandsegmenten innerhalb des polygonalen Ringes aus Außenwand-Leichtbausegmenten angeordnet. Dabei haben die Trennwandsegmente eine Breite b _{T} von mehreren 10 cm, eine Höhe h _{T} von mehreren Metern, die der Höhe h _{A} der Außenwand-Leichtbausegmente entspricht, und eine Dicke d _{T}, die einer Edelstahlblechplattendicke entspricht. Da diese Trennwand mitten in der Biomasse angeordnet ist, kann auf eine Wärmedämmung verzichtet werden. Um jedoch die Trennwand als stabilisierendes Stützelement einzusetzen, weist die Edelstahlblechplatte der Trennwand Sicken, Kantenprofile und Eckenversteifungen auf.

Der Abstand zwischen Trennwand und Außenwand weist mehr als einen Meter auf, so dass eine erste Fermentierungskammer mit scheibenförmigem Grundriss entsteht, die aufgrund des Abstandes bequem bei Wartungs- und Inspektionsarbeiten von dem Prüfpersonal begehbar ist.

Durch die Versteifung der Edelstahlblechplatten kann die Trennwand eine Tragfunktion übernehmen und stützt mindestens zwei entlang der horizontalen Hauptachsen x und y mit der Trennwand mechanisch verbundene horizontale Tragbalken, die auf der Trennwand aufliegen und sich bis auf den polygonalen selbst tragenden Ring erstrecken. Diese Tragbalken können gleichzeitig eine Dachkonstruktion des Fermentierbehälters tragen.

Die Trennwand teilt den Innenraum des Fermentierbehälters in eine scheibenförmige erste Fermentierungskammer zwischen dem polygonalen selbst tragenden Ring und der polygonale ringförmige Trennwand und in eine kreisförmige zweite Fermentierungskammer innerhalb der Trennwand auf.

Die Trennwandsegmente aus nicht rostendem Stahlblech sind zum Boden des Fermentierbehälters hin perforiert und oberhalb eines niedrigsten Füllstandes mit Fermentiersubstanz des Fermentierbehälters gasdicht. Im Bodenbereich der Trennwandsegmente sind eine Mehrzahl der Trennwandsegmente im Bodenbereich mit einer Aussparung versehen. Durch diese Perforierung oder Aussparung im Bodenbereich der Trennwandsegmente kann eine Zwangskonvektion zwischen der Biomasse in der ersten Fermentierungskammer mit der Biomasse in der zweiten Fermentierungskammer erfolgen, indem durch eine geschlossene Abdeckung der ersten Fermentierungskammer ein Gasdruck durch das entstehende Biogas in der ersten Fermentierungskammer auf die Biomasse ausgeübt wird.

In einer weiteren Ausführungsform der Erfindung weist dazu jedes zweite Trennwandsegment im Bodenbereich eine Aussparung auf, die sich über die gesamte Breite eines Trennwandsegmentes erstreckt und eine Höhe von mehreren 10 cm aufweist. Dabei hat sich eine Höhe von etwa 400 mm bewährt, um den oben erwähnten Austausch von Biomasse bzw. eine Konvektion von Biomasse zwischen den beiden Fermentierungskammern sicherzustellen.

In einer weiteren Ausführungsform der Erfindung sind die Trennwandsegmente am Boden des Fermentierbehälters auf der mediendichten mehrere Millimeter dicken EPDM-Folie fixiert. Dabei ragen Befestigungsmittel durch die mediendichte EPDM-Folie hindurch, um die Trennwandsegmente mit der massiven Bodenplatte zu verbinden. Um diese Störung der auf der Bodenplatte liegenden mediendichten EPDM-Folie abzudichten werden die Befestigungsmittel am Boden mit EPDM-Folienhüten versehen. Außerdem können die Trennwandsegmente Verbindungsmuffen für Leitungen aus PVC aufweisen, durch die beispielsweise heißes Wasser zum Aufheizen der Biomasse geführt werden kann, wobei die Leitungen mit Hilfe der Muffen beim Zusammenfügen der Trennwandsegmente zu einer polygonalen Trennwand entstehen.

Weiterhin ist die erste Fermentierungskammer von einer scheibenförmigen segmentierten Abdeckung mediendicht geschlossen. Diese Abdeckung kann auch die kreisförmige zweite Fermentierungskammer mediendicht abdecken, wenn die Außenwand-Leichtbausegmente die gleiche Höhe aufweisen, wie die Trennwandsegmente. Derartige Deckensegmente, die für eine derartige Abdeckung der Fermentierungskammern erforderlich sind, können wie die Außenwand-Leichtbausegmente aufgebaut sein und jeweils eine Wärmedämmplatte mit verzinktem oder lackiertem Stahlblech und außen eine Kunststoffplatte aufweisen. Jedoch wird bei einer optimalen Abdeckung durch Deckensegmente die Innenseite derselben eine Deckschicht aus nicht rostendem Stahl aufweisen und die Oberseite den Leichtbauwandsegmenten entsprechen, die sich durch ihre hohe Wärmeisolation aufgrund der Wärmedämmplatten auszeichnen. Dazu kann der Fermentierbehälter auf seiner Oberseite auf den Hauptachsen angeordnete Tragbalken aufweisen, die die Deckensegmente abstützen und an denen die Deckensegmente fixiert sind. Weiterhin ist es vorgesehen, dass die Längsfugen zwischen den Deckensegmenten in Silikon gelegte Blechabdeckungen aufweisen, so dass diese Längsfugen mediendicht verschlossen bleiben.

In einer weiteren Ausführungsform der Erfindung trägt die Trennwand ein Traggerüst für einen Gasspeicherbehälter, wobei der Gasspeicherbehälter zwischen dem über die Abdeckung hinausragenden Traggerüst in Position gehalten wird. Ein derartiges Traggerüst kann aber auch von der Abdeckung des Fermentierbehälters getragen werden und lediglich an einzelnen Punkten mit der Trennwand verbunden sein. Neben dem Traggerüst für einen Gasspeicherbehälter kann weiterhin ein Aussteifungsrahmen zur Aussteifung unterhalb der Deckensegmente vorgesehen sein, um an dem Aussteifungsrahmen Deckensegmente und Einbauteile des Fennentierbehälters befestigt zu können.

In einer weiteren Ausführungsform der Erfindung ist es vorgesehen, dass die Trennwand von vier vertikalen Stützpfeilern einer Stützstruktur umgeben ist, wobei sich die Stützpfeiler vom Boden des Fermentierbehälters bis oberhalb der ersten scheibenförmigen Abdeckung erstrecken und über horizontale Querträger oberhalb der zweiten Fermentierungskammer miteinander verbunden sind. Diese Stützstruktur kann einerseits die Formstabilität der Trennwand verbessern und andererseits für die Aufnahme des Gasspeicherbehälters dienen.

Das oben erwähnte Traggerüst kann einen Gassack als Gasspeicherbehälter stützen, der auf dem Fermentierbehälter im Bereich der zweiten Fermentierungskammer angeordnet ist und mit dieser über eine Öffnung am Gasspeicherboden verbunden ist. Ein derartiger Gassack kann ebenfalls aus einer EPDM-Folie hergestellt sein und eine Zylinderform mit einem zweischaligen konischen Boden aufweisen. Dieser konische Boden weist die oben erwähnte zentrale Öffnung auf, welche in den Fermentierbehälter hineinragt, wobei die Öffnung gegenüber der Umgebung mediendicht abgedichtet ist. Somit kann Kondenswasser, das sich in dem Gasspeicherbehälter bildet, über die Öffnung in den Fermentierbehälter zurücktropfen.

Vorzugsweise ist in der ersten Fermentierungskammer mindestens ein Rührwerk angeordnet, das die Biomasse ständig in Bewegung hält und eine Zwangskonvektion auch über die Aussparungen oder Perforationen der Trennwandsegmente innerhalb der zweiten Fermentierungskammer aufrecht erhält. Außerdem ist in der zweiten Fementierungskammer mindestens ein Thermo-Gas-Lifter angeordnet, der für eine intensive Durchmischung der Biomasse mit den entstehenden Biogasblasen sorgt und somit eine natürliche Konvektion unterstützt, so dass eventuell auf eine Zwangskonvektion und damit auf einen zusätzlichen Antrieb, beispielsweise für ein Rührwerk, mindestens in der zweiten Fermentierungskammer verzichtet werden kann.

Die Erfindung wird nun anhand der beigefügten Figuren näher erläutert.
Figur 1 zeigt einen schematischen Grundriss eines Fermentierbehälters gemäß einer Ausführungsform der Erfindung;
Figur 2 zeigt eine schematische perspektivische, teilweise aufgeschnittene Ansicht des Fermentierbehälters gemäß Figur 1;
Figur 3 zeigt eine schematische Draufsicht auf den Fermentierbehälter gemäß Figur 1;
Figur 4 zeigt eine schematische Seitenansicht des Fermentierbehälters gemäß Figur 1;
Figur 5 zeigt eine schematische perspektivische teilweise aufgeschnittene Ansicht eines Fermentierbehälters gemäß dem Stand der Technik.

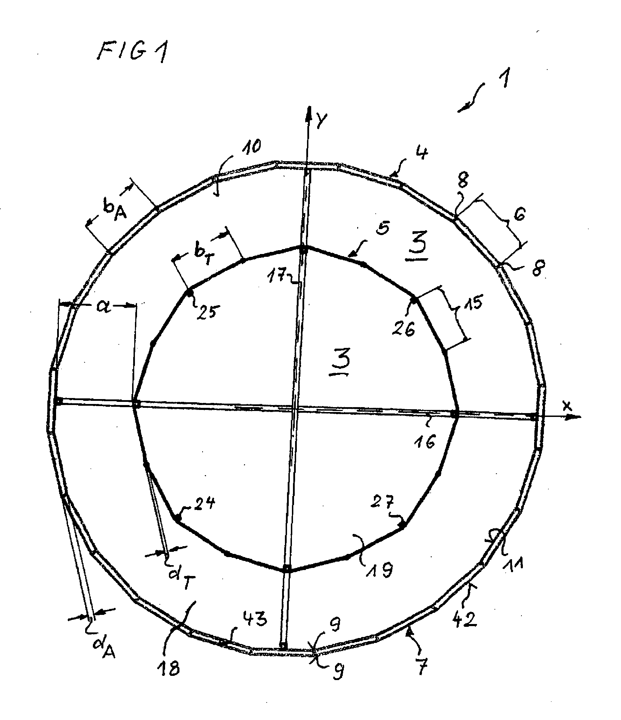

Figur 1 zeigt einen schematischen Grundriss eines Ferinentierbehälters 1 gemäß einer Ausführungsform der Erfindung. Der Fermentierbehälter 1 weist einen Innenraum 3 auf, der zumindest teilweise von mediendichten druckfesten und thermisch isolierenden Wänden 4 umgeben ist. Der Fermentierbehälter 1 weist dazu thermisch isolierende Außenwand-Leichtbausegmente 6 auf, wobei die Außenwand-Leicht-bausegmente 6 einen polygonalen selbst tragenden Ring 7 bilden.

Bei dem in Figur 1 gezeigten Grundriss des Fermentierbehälters 1 ist der Innenraum 3 in eine scheibenförmige erste Fermentierungskammer 18 und eine zweite Fermentierungskammer 19 durch eine Trennwand 5 geteilt. Die Trennwand 5 ist aus einer Vielzahl von Trennwandsegmenten 15 zu einem polygonalen Ring 36 auf einer Bodenplatte 10 zusammengebaut. Die Außenwand-Leichtbausegmente 6 weisen eine deutlich dickere Wandstärke d_{A} auf als die Trennwandsegmente 15 der polygonalen Trennwand 5 mit einer Dicke d _{T}, da die Außenwand-Leichtbausegmente 6 Wärmedämmplatten aus Polyurethan aufweisen, während eine Wärmedämmung für die Trennwand 5 nicht erforderlich ist, da die Trennwand 5 in dem hier gezeigten Grundriss einer Ausführungsform der Erfindung keine Wärme dämmenden Eigenschaften aufweisen muss.

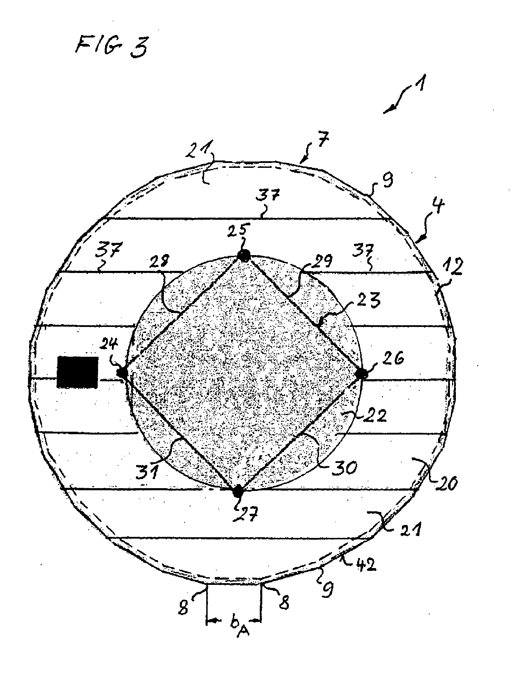

Vielmehr bildet die Trennwand 5 eine Aussteifung und Stützstruktur für den Fermentierbehälter 1 und trägt das Gewicht einer in Figur 3 gezeigten Abdeckung des Fermentierbehälters 1 und zusätzlich das Gewicht und den Stützträger für einen Gasspeicherbehälter, der oberhalb der Abdeckung der Trennwand 5 auf dem Fermentierbehälter 1 angeordnet ist. Ein derartiger Fermentierbehälter 1 wird zum größten Teil in die Erde eingelassen, wozu ein Erdaushub bis zu einer Tiefe von mindestens 2,5 m durchgeführt wird und eine Sauberkeitsschicht aus unbewährtem Beton von einer Dicke zwischen 7 und 10 cm nach dem Erdaushub aufgebracht wird. Eine Drainagefolie und eine Abdichtungsfolie gegen auslaufendes Wasser können auf dem Boden 10 vor dem Einbringen eines Bodenbetons aus mindestens 25 cm dickem Stahlbeton, der gegen Rissbildung armiert ist, aufgelegt werden und nach Aufbringung des Betons und Errichtung der Außenwand-Leichtbausegmente 6 an diesen außenseitig befestigt werden.

Die Außenwand-Leichtbausegmente 6 weisen eine Polyurethan-Wärmedämmplatte auf und sind an den vertikalen Kanten der Ecken 8 des polygonalen Ringes 7 über eine Nut- Federstruktur miteinander verbunden. Diese Leichtbausegmente 6 weisen eine äußere Deckschicht 42 aus verzinktem oder lackiertem Stahlblech mit einer innen liegenden Wärmedämmplatte 43 von mindestens 100 mm Dicke auf.

Die senkrechten Fugen 9 in den Ecken 8 des polygonalen Ringes 7 werden mit Polyurethanschaum ausgeschäumt und mittels Abdeckblechen mit einer Silikonzwischenschicht verschlossen. Zum Schutz der Außenhaut werden die mit Erdreich überdeckten Bereiche der Segmente 6 mit einem Filz abgedeckt und zur besseren Wasserableitung wird auf dem Filz eine Noppenfolie befestigt. Das abfließende Wasser wird in einer den Fermentierbehälter 1 umgebenden Drainageleitung aufgefangen und einem Vorfluter der Biogasanlage zugeleitet.

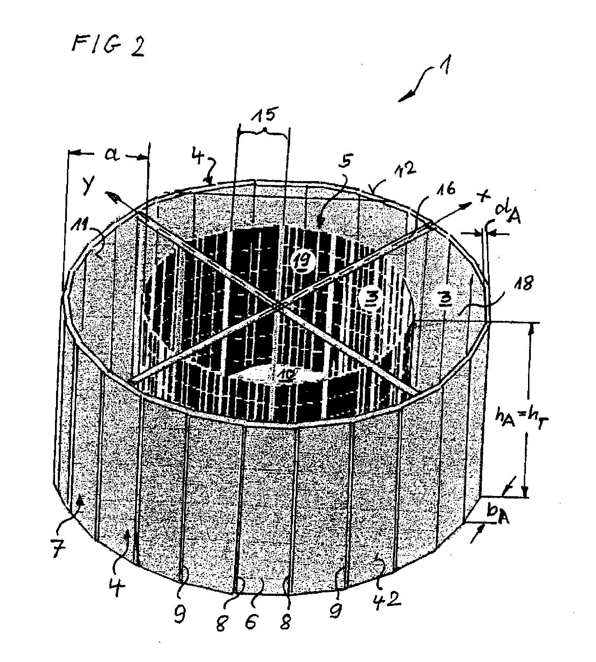

Figur 2 zeigt eine schematische perspektivische, teilweise aufgeschnittene Ansicht des Fermentierbehälters 1 gemäß Figur 1. Diese teilweise aufgeschnittene perspektivische Ansicht zeigt deutlich, dass der Innenraum 3 innerhalb der polygonalen Außenwand 4 keinerlei Stützelemente, Abstandshalter oder Zug- Druckstangen oder ähnliches aufweist, so dass ein derartiger Fermentierbehälter für Wartungs-, Reparatur- oder Inspektionsarbeiten hindernisfrei begehbar ist. Dazu ist ein Abstand a von mindestens 1 m in dieser Ausführungsform der Erfindung zwischen der Außenwand 4 und der inneren polygonalen Trennwand 5 vorgesehen. Die Höhe h _{A} der Außenwand-Leichtbausegmente 6 ist gleich der Höhe h _{T} der inneren Trennwand 5. Das hat den Vorteil, dass durchgängige Deckensegmente zum Abdecken des Fennentierbehälters 1 auf die Wandsegmente aufgelegt werden können. Um eine derartige Abdeckung, wie sie in Figur 3 gezeigt wird, zu stützen und zu tragen sind, in den Hauptachsen x und y auf die ringförmigen Wandstrukturen Tragbalken 16 und 17 aufgelegt, die durch in dieser Figur nicht gezeigte vertikale Stützpfeiler zusätzlich gestützt werden können.

Vor dem Einbringen der inneren Trennwand 5 erhält der Fermentierbehälter 1 eine Auskleidung auf dem Boden liegend und an der Innenfläche 11 der Außenwand-Leichtbausegmente 6 aus einer EPDM-Folie, welche an einem Wand-Deckenübergang mit einem Winkel, mit dem auch Deckensegmente fixiert werden, befestigt wird. Als Montagehilfe und zur zusätzlichen Abdichtung wird diese EPDM-Folie an den Innenflächen 11 der Außenwand-Leichtbausegmente 6 mit einem geeigneten Kleber fixiert. Dazu ist die EPDM-Folie dieser Auskleidung zwischen 3 und 5 mm dick und aufgrund ihrer oben erwähnten Eigenschaften für einen Fermentierbehälter 1 besonders gut geeignet. Durchdringungen durch die Folie, wie sie beispielsweise zum Fixieren der Trennwandsegmente 15 auf der mit der Auskleidung beschichteten Bodenplatte 10 vorzusehen sind, werden zusätzlich mittels Flanschaufsätzen und/oder EPDM-Folienhüten abgedichtet. Durchdringungen, wie sie für Heißwasserleitungen sowie für den Zu- und Abfluss von Biomasse in den Fermentierbehälter erforderlich sind, werden zusätzlich mit Klemmringen an den Rohren und Stützen mechanisch gegen ein Abrutschen gesichert.

Die Trennwandsegmente 15 sind in dieser Ausführungsform 1,15 m breit und in einem Abstand a von 1,5 m von dem polygonalen Außenring 7 angeordnet. Oberhalb eines niedrigsten Füllstandes für den Fermentierbehälter 1 sind die Trennwandsegmente 15 gasdicht ausgeführt und unterhalb weisen die Trennwandsegmente 15 im Bodenbereich Aussparungen mit einer Höhe von ca. 400 mm auf, wobei jedes zweite Trennwandsegment 15 eine derartige Aussparung für seine gesamte Breite in dieser Ausführungsform der Erfindung aufweist.

In den Hauptachsen x und y ist ein Aussteifungsrahmen im Bereich der Trennwand 5 eingezogen. Dieser Aussteifungsrahmen übernimmt einerseits die Aussteifung und andererseits die Befestigung von Einbauteilen wie Thermo-Gas-Lifter, Rührwerk und die Befestigung der Deckensegmente der Abdeckung. Dabei dienen senkrechte Segmentaussteifungen zur Stabilität der Trennwandsegmente 15. Eine Befestigung dieser Trennwandsegmente am Boden erfolgt mit geeigneten Befestigungsmaterialien, wie Dübeln und Schrauben, und die mittels EPDM-Folienhüte zusätzlich abgedichtet werden. In einige der Segmente 6 und/oder 15 werden Verbindungsmuffen für die Verbindung von Leitungen, beispielsweise für den Vorlauf und den Rücklauf der Biomasse oder für Heizleitungen eingearbeitet. Alle Leitungen bis auf eine Gärgutausbringleitung werden unterhalb des niedrigsten Pegelstandes durch die Außenwand-Leichtbausegmente 6 durchgeführt. Soweit es Durchführungen in der Trennwand 5 zwischen der ersten Fermentierungskammer 18 und der zweiten Fermentierungskammer 19 betrifft, werden diese mittels verschweißten Schraubverbindungen hergestellt. Die oben erwähnten Leitungen sind aus einem Kunststoff, wie PVC (Polyvinylchlorid) oder aus nicht rostendem Stahl hergestellt.

Figur 3 zeigt eine schematische Draufsicht auf den Fermentierbehälter 1 gemäß Figur 1. Diese Draufsicht zeigt im Wesentlichen den Deckenaufbau, der aus Deckensegmenten 21 zusammengesetzt ist. Diese Deckensegmente 21 sind ebenfalls wie die Außenwand-Leichtbausegmente aufgebaut und können optional auch mit einem nicht rostenden Stahlblech innen als Abdeckung und einem Kunststoff außen hergestellt sein. Diese Deckensegmente 21 werden auf die W andsegmente aufgelegt und auf diesen befestigt. Von den oben erwähnten in den Hauptachsen x und y verlaufenden Tragbalken werden sie gestützt und gegen Abheben gesichert. Die Längsfugen 37 zwischen den Nut-Federverbindungen der Deckensegmente 21 sind beidseitig durch in Silikon gelegte Blechabdeckungen mediendicht verschlossen.

Die W and-Deckenverbindung 12 wird innen mittels Edelstahlwinkeln ausgeführt. Die Befestigung der Winkel erfolgt mit Schrauben aus nicht rostendem Stahl. Die Edelstahlwinkel werden vor der Montage mittels geeignetem Kleber an der auskleidenden EPDM-Folie des Fermentierbehälters 1 angeklebt und dann mit den Außenwand-Leichtbausegmenten 6 verschraubt. Die Abstände dieser mechanischen Befestigungen durch Edelstahlwinkel betragen in dieser Ausführungsform der Erfindung beispielsweise 30 cm. Die Übergangsfuge wird zwischen Wand- und Deckensegment mit Polyurethan-Schaum aufgefüllt und auf der Außenseite wird ein Blechwinkel zur Abdeckung der Deckensegmente 21 und zur zusätzlichen Stützung dieser mit PU-Schaum aufgefüllten Fuge angebracht. Bei einer Montage dieses äußeren ringförmig und horizontal verlaufenden Abdeckwinkels wird dieser mit einer Silikonschnur auf beiden Schenkeln des Winkels aufgebracht, um die Witterungsbeständigkeit und Dichtigkeit zu verbessern. Die mechanische Fixierung des horizontalen Abdeckwinkels zwischen Wand- und Deckensegment weist Edelstahlschrauben auf.

Außerdem zeigt Figur 3 die Position und Größe des auf die Abdeckung des Fermentierbehälters 1 in einem Bereich der inneren Trennwand aufgebrachten Gassacks als Gasspeicherbehälter 22. Dieser Gasspeicherbehälter 22 wird von einem Traggerüst 23 aus Aluminiumprofilen zwischen vertikalen Stützpfeilern 24 bis 27 gehalten, die miteinander über horizontale Querträger 28 bis 31, wie es Figur 3 zeigt, verbunden sind.

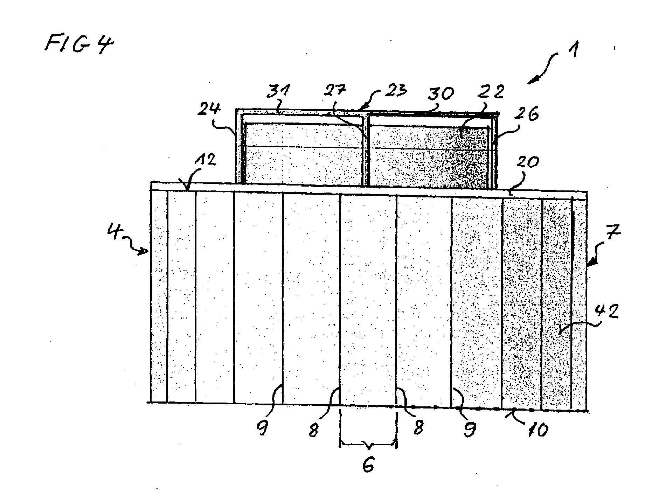

Figur 4 zeigt eine schematische Seitenansicht des Fermentierbehälters 1 gemäß Figur 1. Diese perspektivische Ansicht zeigt eine Gesamtansicht des Fermentierbehälters 1. aus einem polygonalen Ring 7 mit Außenwand-Leichtbausegmenten 6 und Deckensegmenten einer Abdeckung 20 und den Gasspeicherbehälter 22 im Bereich der inneren Trennwand. Dieser Gasspeicherbehälter 22 besteht aus einer EPDM-Folie, die werkseitig in der Größe und Form für den jeweiligen Fermentierbehälter 1 angeliefert wird. Die Größe des Gasspeicherbehälters 22 beträgt in dieser Ausführungsform der Erfindung 25 m³ und ist in Zylinderform ausgebildet. Der in der Figur nicht sichtbare Boden des Gasspeicherbehälters 22 ist kegelförmig und zweischalig mit einem eingearbeitetem Kunststoffkegel, welcher eine Wasserbildung im Boden des Gasbehälters verhindert, das durch eine Öffnung im Boden zu dem Fermentierbehälter abtropfen kann.

Diese Öffnung im Boden des Gasspeicherbehälters 22 ist mediendicht gegenüber der Umgebung abgedichtet, indem der Gasspeicherbehälter 22 mit den Deckensegmenten gasdicht verbunden ist. Die Höhe des Gasspeicherbehälters 22 beträgt in dieser Ausführungsfonn der Erfindung 1000 mm und ist von umlaufenden Querködern unterteilt. An diesen Köderelementen werden Schlaufen und Ösen zur Stabilisierung und Befestigung von einem Traggerüst 23 angebracht. Das Traggerüst 23 des Gasspeicherbehälters 22 besteht in dieser Ausführungsform der Erfindung aus Aluminium-Leichtbauprofilen. Diese Aluminium-Leichtbauprofile werden auf der Abdeckung 20 des Fermentierbehälters 1 befestigt.

Die Windkräfte werden auf die in dem Fermentierbehälter 1 liegende Tragkonstruktion abgeleitet. Zum Schutz gegen Witterungseinflüsse wird auf dem Traggerüst ein hier nicht gezeigtes Trapezblechdach zur Vermeidung von Beschädigungen montiert. Das Trapezblechdach erhält dazu eine leichte Neigung in Satteldachform. Eine Gasleitung wird mittels Foliendurchdringung an den Gasspeicherbehälter angeschlossen und zu dem Konsumenten geführt. Oberhalb des Gasspeichers ist ein Drucksensor für die Überprüfung des Gasdrucks im Speicher installiert. Außerdem ist in die EPDM-Folie ein Bullauge zur visuellen Kontrolle eingelassen.

Anstelle eines Gasspeichers und einer in dieser Ausführungsform der Erfindung gezeigten Abdeckung kann auch eine topfartiger Abdeckhaube über den Fermentierbehälter 1 mit seiner polygonalen Außenwand 4 gestülpt und in ein Wasserschloss getaucht werden. Eine derartige Wasserschlosskonstruktion hat den Vorteil, dass sich das Volumen des Gasspeicheibehälters dem Biogasdruck durch Heben und Senken der haubenförmigen Abdeckung in dem Wasserschloss anpasst. Dabei ist es von Vorteil, diese haubenförmige Abdeckung wiederum aus einer EPDM-Folie zu bilden. Eine weitere alternative Ausführung besteht darin, das Zweikammmersystem beizubehalten und lediglich die erste Kammer mit Deckensegmenten gasdicht zu verschließen und die zweite Kammer mit einem vollflächigen Ausschnitt für den Gasaustritt zu versehen. Über den gesamten Fermentierbehälter wird dann eine topfartiger Abdeckhaube aus einer EPDM-Folie gestülpt und von einem Wasserschloss umgeben, das die topfartiger Abdeckhaube gasdicht zur Umgebung verschlossen hält.

Figur 5 zeigt eine schematische perspektivische, teilweise aufgeschnittene Ansicht eines Fermentierbehälters gemäß dem Stand der Technik, wie er bereits eingangs beschrieben ist, so dass sich eine Wiederholung an dieser Stelle erübrigt.

### [54] Bezugszeichenliste

1 Fermentierbehälter (Ausführungsform)
2 Fermentierbehälter (Stand der Technik)
3 Innenraum
4 Wand
5 Trennwand
6 Außenwand-Leichtbausegment
7 polygonaler selbst tragender Ring
8 Ecken des polygonalen Ringes
9 senkrechte Fuge
10 Bodenplatte
11 Innenfläche des Leichtbausegments
12 Wand-Deckenübergang
15 Trennwandsegment
16 Tragbalken (x-Richtung)
17 Tragbalken (y-Richtung)
18 erste Fermentierungskammer
19 zweite Fermentierungskammer
20 Abdeckung
21 Deckensegment
22 Gasspeicherbehälter
23 Traggerüst
24 Stützpfeiler
25 Stützpfeiler
26 Stützpfeiler
27 Stützpfeiler
28 Querträger
29 Querträger
30 Querträger
31 Querträger
32 Innenaufbau
33 Außenwand
34 Außenwand
35 Außenwand
36 polygonaler Ring
37 Längsfugen der Deckensegmente
38 Paneel
39 Lasche
40 Diagonalstrebe
41 Zug- und Druckstange
42 äußere Deckschicht
43 Wännedämmplatte
a Abstand zwischen Außenwand und Trennwand
b _{A} Breite der Außenwand-Leitbausegmente
h _{A} Höhe der Außenwand-Leitbausegmente
d _{A} Dicke der Außenwand-Leitbauelemente
b _{T} Breite der Trennwandsegmente
h _{T} Höhe der Trennwandsegmente
d _{T} Dicke der Trennwandsegmente
x horizontale Hauptachse
y horizontale Hauptachse

## Patentansprüche

1. Fermentierbehälter für eine Biogasanlage, dessen Innenraum (3) zumindest teilweise von mediendichten, druckfesten und thermisch isolierenden Wänden (4) umgeben ist, wobei
- der Fermentierbehälter (1) thermisch isolierende Außenwand-Leichtbausegmente (6) aufweist, die einen polygonalen selbst tragenden Ring bilden;
- eine polygonale ringförmige Trennwand (5) aus Trennwandsegmenten (15) innerhalb des polygonalen Ringes (7) aus Außenwand-Leichtbausegmenten (6) angeordnet ist;
- die Trennwand (5) den Innenraum (3) des Fermentierbehälters (1) in eine scheibenförmige erste Fermentierungskammer (18) zwischen dem polygonalen selbst tragenden Ring (7) und der polygonale ringförmige Trennwand (5) und eine kreisförmige zweite Fermentierungskammer (19) innerhalb der Trennwand (5) aufteilt;
- eine Mehrzahl der Trennwandsegmente (15) im Bodenbereich eine Aussparung aufweisen.

2. Fermentierbehälter Anspruch 1,
**dadurch gekennzeichnet, dass**
die Ecken (8) des polygonalen Ringes (7) vertikal angeordnete Nut-Federübergänge der Wärmedämmplatten aufweisen.

3. Fermentierbehälter nach Anspruch 2,
**dadurch gekennzeichnet, dass**
senkrechte Fugen (9) der Nut-Federübergänge der Wärmedämmplatten an den Ecken (8) des polygonalen Ringes (7) mit Polyurethan-Schaum aufgefüllt und mittels Abdeckblechen und einer Silikonzwischenschicht mediendicht geschlossen sind.

4. Fermentierbehälter nach einem der vorhergehenden Ansprüche,
**dadurch gekennzeichnet, dass**
die Außenwand-Leichtbausegmente (6) als polygonaler selbst tragender Ring (7) auf einer Bodenplatte (10) unterhalb der Geländeoberfläche angeordnet ist.

5. Fermentierbehälter nach einem der Ansprüche 1 bis 4,
**dadurch gekennzeichnet, dass**
die Trennwandsegmente (15) nicht rostende Stahlbleche aufweisen, die zum Boden (10) des Fermentierbehälters (1) hin perforiert sind und oberhalb eines niedrigsten Füllstandes mit Fermentiersubstanz des Fermentierbehälters gasdicht sind.

6. Fermentierbehälter nach einem der Ansprüche 1 bis 5,
**dadurch gekennzeichnet, dass**
jedes zweite Trennwandsegment (15) im Bodenbereich eine Aussparung aufweist, die sich über die gesamte Breite eines Trennwandsegmentes (15) erstreckt und eine Höhe von mehreren 10 cm aufweist.

7. Fermentierbehälter nach einem der Ansprüche 1 bis 6,
**dadurch gekennzeichnet, dass**
die Trennwand (5) ein Traggerüst (23) für einen Gasspeicherbehälter (22) trägt, wobei der Gasspeicherbehälter (22) zwischen dem über die Abdeckung hinausragenden Traggerüst (23) in Position gehalten wird.

8. Fermentierbehälter nach einem der Ansprüche 1 bis 6,
**dadurch gekennzeichnet, dass**
das Traggerüst (23) einen Gassack als Gasspeicherbehälter (22) stützt, der auf dem Fermentierbehälter (1) im Bereich der zweiten Fermentierungskammer (19) angeordnet ist und mit dieser über eine Öffnung am Gasspeicherboden verbunden ist.

9. Fermentierbehälter nach Anspruch 8,
**dadurch gekennzeichnet, dass**
der Gasspeicherbehälter (22) eine Zylinderform und einen zweischaligen konischen Boden aufweist.

10. Fermentierbehälter nach Anspruch 9,
**dadurch gekennzeichnet, dass**
der konischen Boden eine zentrale Öffnung aufweist, welche in den Fermentierbehälter hineinragt und wobei die Öffnung gegenüber der Umgebung mediendicht abgedichtet ist.

## Claims

1. Digester tank for a biogas plant, having an interior (3) which is at least partially surrounded by media-tight, pressure-tight and thermally insulating walls (4), wherein
- the digester tank (1) comprises thermally insulating light-weight exterior wall segments (6) which form a self-supporting polygonal ring;
- a polygonal annular partition (5) assembled from partition segments (15) is placed within the polygonal ring (7) of light-weight exterior wall segments (6);
- the partition (5) divides the interior (3) of the digester tank (1) into a disc-shaped first digester chamber (18) between the self-supporting polygonal ring (7) and the polygonal annular partition (5) and a circular second digester chamber (19) within the partition (5);
- a majority of the partition segments (15) is provided with a cut-out in the bottom region.

2. Digester tank according to claim 1, **characterised in that** the corners (8) of the polygonal ring (7) comprise vertically arranged groove-to-tongue transitions of the heat-insulating boards.

3. Digester tank according to claim 2, **characterised in that** vertical gaps (9) of the groove-to-tongue transitions of the heat-insulating boards at the corners (8) of the polygonal ring (7) are filled with polyurethane foam and provided with a media-tight seal by means of cover plates and a silicone intermediate layer.

4. Digester tank according to any of the preceding claims, **characterised in that** the light-weight exterior wall segments (6) are placed as a self-supporting polygonal ring (7) on a base plate (10) below the site surface.

5. Digester tank according to any of claims 1 to 4, **characterised in that** the partition segments (15) comprise stainless steel plates which are perforated towards the bottom (10) of the digester tank (1) and gas-tight above a minimum fermenting substance level of the digester tank (1).

6. Digester tank according to any of claims 1 to 5, **characterised in that** every other partition segment (15) has a cut-out in the bottom region, which extends across the entire width of the partition segment (15) and has a height of several 10 cm.

7. Digester tank according to any of claims 1 to 6, **characterised in that** the partition (5) supports a support rack (23) for a gas reservoir (22), the gas reservoir (22) being held in position between the support rack (23), which projects beyond the cover.

8. Digester tank according to any of claims 1 to 6, **characterised in that** the support rack (23) supports a gas bag acting as a gas reservoir (22), which is located in the digester tank (1) in the region of the second digester chamber (19) and connected thereto by an opening in the gas reservoir bottom.

9. Digester tank according to claim 8, **characterised in that** the gas reservoir (22) has a cylindrical shape and a double-shell conical bottom.

10. Digester tank according to claim 9, **characterised in that** the conical bottom has a central opening which projects into the digester tank, the opening being provided with a media-tight seal against the environment.

## Revendications

1. Contenant de fermentation destiné à une installation de biogaz, dont l'espace intérieur (3) est entouré au moins partiellement de parois étanches aux fluides, résistantes à la pression et thermiquement isolantes,
- le contenant de fermentation (1) comportant des segments légers (6) de paroi extérieure thermiquement isolants qui forment un anneau (7) polygonal autoportant;
- une paroi de séparation (5) polygonale annulaire à partir de segments de paroi de séparation (15) étant disposée à l'intérieur de l'anneau polygonal (7) à partir des segments légers (6) de paroi extérieure;
- la paroi de séparation (5) séparant l'espace interne (3) du contenant de fermentation (1) en une première chambre de fermentation (18) discoïde entre l'anneau (7) polygonal autoportant et la paroi de séparation (5) polygonale annulaire et en une seconde chambre de fermentation circulaire à l'intérieur de la paroi de séparation (5);
- une pluralité des segments de paroi de séparation (15) présentant dans la zone du fond un évidemment.

2. Contenant de fermentation selon la revendication 1, caractérisé en ce queles angles (8) de l'anneau polygonal (7) comportent des liaisons languette - rainure des plaques d'isolation thermique, lesdites liaisons étant disposées verticalement.

3. Contenant de fermentation selon la revendication 2 **caractérisé en ce que** des fentes verticales (9) des liaisons languette - rainure des plaques d'isolation thermique sont remplies de mousse polyuréthane dans les angles (8) de l'anneau polygonal (7) et sont fermées de manière étanche aux fluides au moyen de tôles de recouvrement et d'une couche intermédiaire de silicone.

4. Contenant de fermentation selon l'une quelconque des revendications précédentes, caractérisé en ce queles segments légers (6) de paroi extérieure en tant qu'anneau (7) polygonal autoportant sont disposés sur une plaque de fond (10) sous la surface du terrain.

5. Contenant de fermentation selon l'une quelconque des revendications 1 à 4, **caractérisé en ce que** les segments de paroi de séparation (15) présentent des tôles d'acier inoxydable, qui sont perforées vers le fond (10) du contenant de fermentation et au-dessus d'un niveau de remplissage le plus bas sont rendues étanches au gaz au moyen d'une substance de fermentation du contenant de fermentation.

6. Contenant de fermentation selon l'une quelconque des revendications 1 à 5, **caractérisé en ce qu'**un segment de paroi de séparation (15) sur deux dans la région de fond présente un évidemment, qui s'étend sur toute la largeur d'un segment de paroi de séparation (15) et dont la hauteur est de plusieurs dizaines de centimètres.

7. Contenant de fermentation selon l'une quelconque des revendications 1 à 6, **caractérisé en ce que** la paroi de séparation (5) porte un bâti porteur (23) destiné à un contenant de stockage de gaz (22), le contenant de stockage de gaz (22) étant maintenu en position entre le bâti porteur (22) faisant saillie du recouvrement.

8. Contenant de fermentation selon l'une quelconque des revendications 1 à 6, caractérisé en ce quele bâti porteur (23) soutient un sac gazeux en tant que contenant de stockage de gaz (22) qui est disposé sur le contenant de fermentation (1) dans la région de la seconde chambre de fermentation (19) et est relié à ladite seconde chambre de fermentation par une ouverture dans le fond du contenant de stockage de gaz.

9. Contenant de fermentation selon la revendication 8 **caractérisé en ce que** le contenant de stockage de gaz (22) présente une forme cylindrique et un fond conique à double paroi.

10. Contenant de fermentation selon la revendication 9 **caractérisé en ce que** le fond conique présente une ouverture centrale laquelle débouche dans le contenant de fermentation et l'ouverture étant rendue étanche aux fluides de l'environnement.
